# EUROPEAN PATENT APPLICATION

(11) **EP 4 851 137 A1**
(43) Date of publication of application: **09.12.2026**
(21) Application number: 25749233.0
(22) Date of filing: 31.01.2025
(51) Int. Cl.: C07D 209/18, A61K 49/00, C07H 15/26, C07D 475/02

(54) **COMPOSITION FOR CANCER DIAGNOSIS TARGETING TUMOR AND/OR TUMOR MICROENVIRONMENT AND USE THEREOF**

(30) Priority: 30.01.2024 US 202463626971 P; 09.12.2024 KR 20240181865; 24.01.2025 US 202518831452
(71) Applicant: Korea University Research and Business Foundation, Seoul 02841 (KR)
(72) Inventor: KIM, Hyun Koo, Seoul 06579 (KR); RHO, Ji-Yun, Seoul 08096 (KR); JEON, Ok Hwa, Seoul 08308 (KR); KIM, Kyungsu, Seoul 03483 (KR); CHOI, Byeong Hyeon, Seoul 08529 (KR); CHOI, Hak Soo, Needham, Massachusetts 02494 (US); BAO, Kai, Harvard, Massachusetts 01451 (US)
(74) Representative: ZBM Patents - Zea, Barlocci & Markvardsen
(86) International application number: PCT/KR2025/099159
(87) International publication number: WO 2025/165194

(57) **Abstract**

The present invention relates to a composition for cancer diagnosis targeting a tumor and/or a tumor microenvironment and use thereof and, more specifically, provides a fluorescent contrast agent composition targeting biomolecules overexpressed in cancer cells and a tumor microenvironment. According to the present invention, a fluorescent contrast agent capable of targeting both cancer (folate receptor alpha-expressing cancer) cells and tumor-related macrophages in a tumor microenvironment can be provided.

## Description

### TECHNICAL FIELD

The present disclosure relates to a composition for cancer diagnosis targeting a tumor and/or tumor microenvironment and a use thereof, and more specifically, to a composition for cancer diagnosis targeting a substance overexpressed in a tumor and/or cancer microenvironment and a use thereof.

### BACKGROUND ART

The tumor microenvironment (TME) refers to a totality composed of complex and diverse elements such as other cells surrounding cancer cells, extracellular matrix, growth hormones, and signaling substances. The cancer microenvironment plays an important role as a culture medium for seeds called cancer cells to grow.

Among the various immune cells that gather at a tumor site, macrophages, in particular, are cells that appear abundantly throughout the entire process of cancer development, and have been shown to play a role in promoting cancer in clinical studies and studies using mice. The macrophages stimulate angiogenesis of a tumor and increase the invasion, migration ability, and intravascular invasion of cancer cells. During the metastasis process, macrophages prepare the metastatic site and promote extravascular escape, survival, and continued growth of cancer cells. These series of cancer-promoting activities are caused by distinguishable subpopulations of macrophages, and in conclusion, the macrophages may be important targets for cancer treatment.

In relation to the role of tumor-associated macrophages in the cancer microenvironment, research on cancer biomarkers and therapeutic targets is in progress. It has been reported that tumors are promoted by tumor-associated macrophages in various cancer tissues, and that the more tumor-associated macrophages present, the lower the survival rate of cancer patients and the worse the prognosis. Accordingly, an approach targeting tumor-associated macrophages is regarded as a new strategy to treat cancer. Accordingly, tumor-associated cells may not only serve as important diagnostic and prognostic markers for tumors, but may also be therapeutic targets. When performing angiography targeting tumor-associated macrophages as biomarkers, during surgery, the location of the tumor and the interface between cancer and normal tissue may be imaged in real time.

Conventionally, as the importance of cancer detection images during surgery has increased, various near-infrared fluorescent contrast agents capable of passive or active targeting have been developed. However, passive targeting using ICG has low fluorescence under near-infrared light and binds to albumin in the blood when injected *in vivo*, and is quickly eliminated in the liver, and thus cancer detection efficiency is low. In the case of active targeting, the issue of stability in the human body has not yet been resolved. Most of the existing targeted fluorescent contrast agents are synthesized targeting proteins that are presently widely known to be overexpressed in cancer, and therefore rely on research to identify the mechanism of a specific protein. However, in comparison, the cancer microenvironment, which refers to the surrounding environment of cancer, is composed of various immune cells regardless of the type or mutation of the cancer and maintains a relatively similar environment. Accordingly, if a fluorescent contrast agent that may target the cancer microenvironment is developed, it is expected to be usable for general purposes regardless of the type or mutation of cancer.

### [Prior Art Document]

### [Patent Document]

KR Patent Application Publication No. 10-2020-0027895

### [Non-Patent Document]

Yang C et al., ZW800-PEG: A Renal Clearable Zwitterionic Near-Infrared Fluorophore for Potential Clinical Translation. Angew Chem Int Ed Engl. 2021 Jun 14;60(25):13847-13852.

### DISCLOSURE OF THE INVENTION

### TECHNICAL GOALS

The present disclosure relates to a composition for cancer diagnosis targeting a tumor and/or tumor microenvironment and a use thereof, and more specifically, to a composition for cancer diagnosis targeting folate receptor alpha on cancer cells, folate receptor beta of tumor-associated macrophages, and/or mannose receptor on tumor-associated macrophages.

The aspects of the present disclosure are not limited to those mentioned above, and other aspects not mentioned herein will be clearly understood by those skilled in the art from the following description.

### TECHNICAL SOLUTIONS

An embodiment of the present disclosure may provide a compound represented by [Formula 1] below or a pharmaceutically acceptable salt thereof: 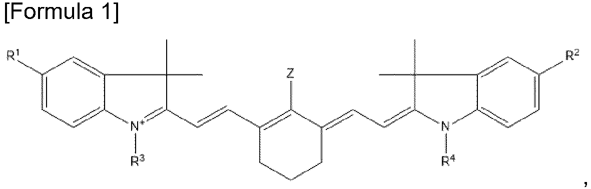
in which R¹ and R² are the same as or different from each other, and are each independently any one or more selected from the group consisting of hydrogen, a hydroxy group, a C₁-C₅ alkoxy group, (Y)ₘX, (Y)ₘCOO⁻, (Y)ₘSO₃⁻, (Y)ₘPO₃H⁻, and combinations thereof;
R³ and R⁴ are the same as or different from each other, and are each independently any one or more selected from the group consisting of (Y)ₙH, (Y)ₙ(CO)R⁵, and combinations thereof;
R⁵ is a hydroxy group or 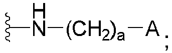
A is a C₂-C₆ heterocycloalkyl group containing any one or more heteroatoms selected from the group consisting of substituted or unsubstituted N, O, and S;
X is a halogen group,
Y is CH₂ or CH₂CH₂O;
Z is halogen or 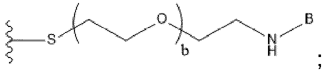
B is hydrogen or 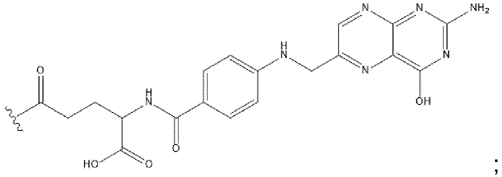 and
a, b, m, and n may be the same as or different from each other, and may each independently be an integer from 0 to 5.

In an embodiment of the present disclosure, in Formula 1, R¹ and R² may each independently be hydrogen or (Y)ₘSO₃⁻.

In another embodiment of the present disclosure, in Formula 1, R³ and R⁴ may each independently be (Y)ₙ(CO)R⁵;
R⁵ may be a hydroxyl group or 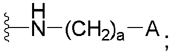 and
A may be a C₂-C₆ heterocycloalkyl group containing one or more heteroatoms selected from the group consisting of substituted or unsubstituted N, O, and S.

In another embodiment of the present disclosure, the A may be

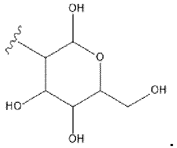

In yet another embodiment of the present disclosure, in Formula 1, Z may be Cl,

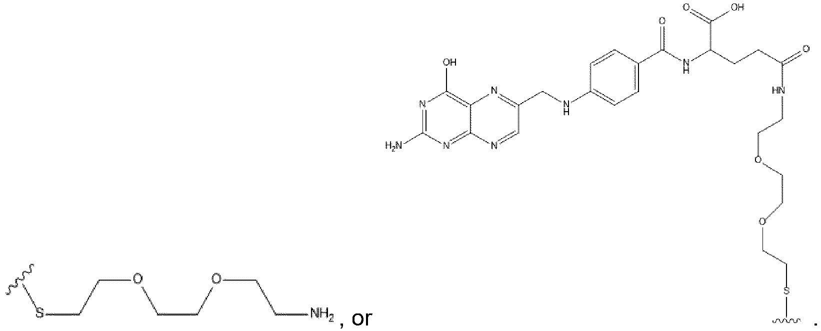

In yet another embodiment of the present disclosure, the compound may be any one or more selected from the group consisting of compounds represented by the following [Formula 1-1] to [Formula 1-6]: 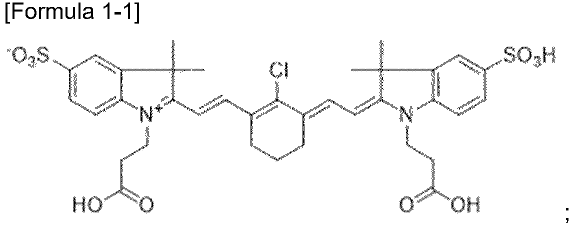 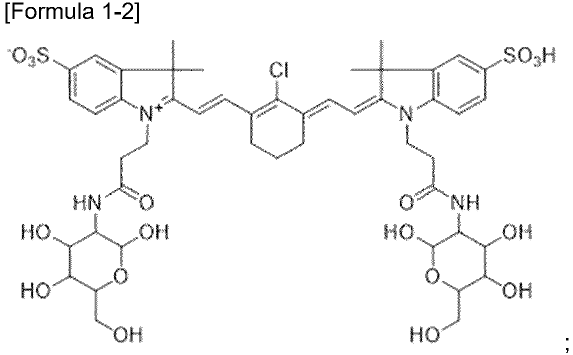 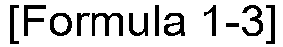 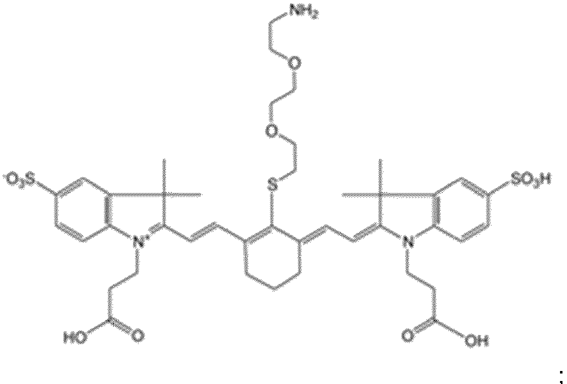 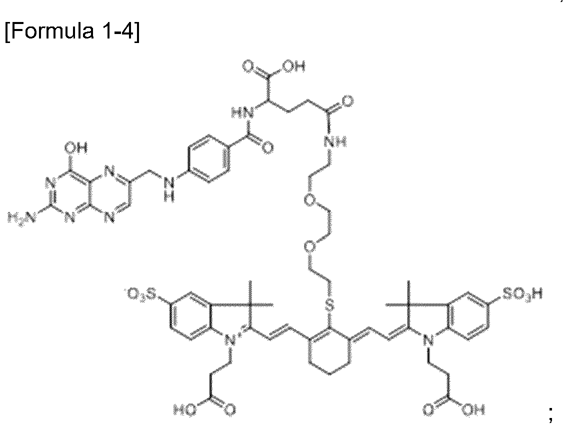 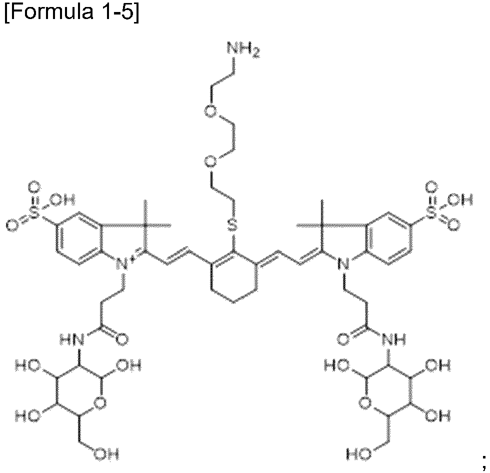 and

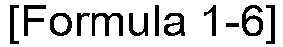

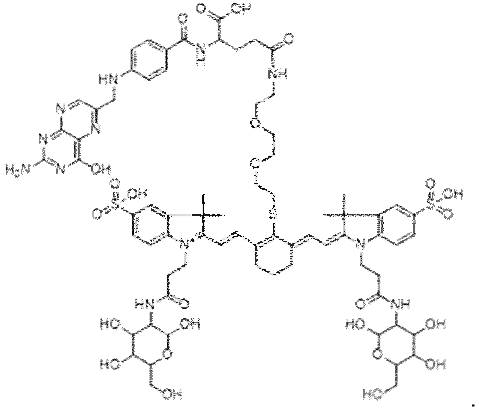

In addition, an embodiment of the present disclosure may provide a contrast agent composition for cancer diagnosis including the compound or a pharmaceutically acceptable salt thereof as an active ingredient.

In an embodiment of the present disclosure, the contrast agent composition may target a tumor and/or tumor microenvironment.

In another embodiment of the present disclosure, the contrast agent composition may bind to any one or more of folate receptor alpha of cancer cells, folate receptor beta of tumor-associated macrophages (TAMs) in the tumor microenvironment, and CD206 of TAM.

In yet another embodiment of the present disclosure, the contrast agent composition may simultaneously target the tumor and the tumor microenvironment.

In addition, an embodiment of the present disclosure provides a method for cancer diagnosis including targeting a tumor and/or tumor microenvironment using any one of the compositions.

### EFFECTS OF THE INVENTION

An embodiment of the present disclosure relates to a composition for cancer diagnosis or cancer imaging targeting tumor-associated macrophages in the cancer microenvironment, including folic acid and a fluorescent substance, and more specifically, to a fluorescent contrast agent composition targeting folate receptor alpha in tumor cells and/or folate receptor beta in tumor-associated macrophages as a biomarker. According to an embodiment of the present disclosure, it is possible to provide a fluorescent contrast agent capable of reacting more specifically than a fluorescent contrast agent that reacts to temperature, pH, hypoxia, and the like which are characteristics of a simple cancer microenvironment, and specifically targeting tumors (folate receptor alpha-expressing tumors) and tumor-associated macrophages within the tumor microenvironment.

### BRIEF DESCRIPTION OF THE DRAWINGS

FIG. 1 shows the absorbance and fluorescence analysis results of Compound 1;
FIG. 2 shows the absorbance and fluorescence analysis results of Compound 2;
FIG. 3 shows the absorbance and fluorescence analysis results of Compound 4;
FIG. 4 shows the absorbance and fluorescence analysis results of Compound 6;
FIG. 5A shows the results of fluorography of the compounds of the present disclosure depending on the expression of folate receptors;
FIG. 5B shows the fluorescence intensity of fluorography experiment;
FIG. 6 shows cell viability when two lung cancer cell lines, A549 and H522, were treated with compound 6 (FMK-2) at different concentrations;
FIG. 7A shows the mouse model experiment fluorescence results of Compound 6 of the present disclosure;
FIG. 7B shows the ratio of near-infrared fluorescence signal in tumor compared to normal tissue;
FIG. 8 shows frozen slices of tissue administered with Compound 6 of the present disclosure to a mouse model, followed by near-infrared fluorescence analysis, H&E staining, and immunohistochemical analysis;
FIG. 9 shows the results of fluorescence detection after injecting the compound of the present disclosure into a mouse and extracting the organ;
FIG. 10 is a half-life graph of FMK-2;
FIG. 11 shows the results of fluorescence analysis identified after intravenous injection of FMK-2 into a mouse inflammation model;
FIG. 12 shows the results of a comparative staining experiment of ZW800-PEG and human lung cancer cell line;
FIG. 13 shows the results of fluorescence imaging of cancer tissue after intravenous injection of OCTL14 and FMK-2 into a mouse cancer model;
FIG. 14 shows a schematic diagram illustrating the tumor microenvironment; and
FIG. 15 is a diagram illustrating the mechanism by which a composition for cancer imaging prepared according to an embodiment of the present disclosure expresses fluorescence.

### BEST MODE FOR CARRYING OUT THE INVENTION

Hereinafter, embodiments will be described in detail with reference to the attached drawings. However, various changes may be made to the embodiments, so the scope of the patent application is not limited or restricted by these embodiments. It should be understood that all changes, equivalents, or substitutes for the embodiments are included in the scope of right.

The terms used in the present specification are merely used to describe specific embodiments and are not intended to limit the present disclosure. A singular expression includes a plural expression, unless the context clearly states otherwise. In the present specification, it should be understood that the terms such as "include" or "have" are merely intended to indicate that features, numbers, steps, operations, components, parts, or combinations thereof are present, and are not intended to exclude the possibility that one or more other features, numbers, steps, operations, components, parts, or combinations thereof will be present or added.

Unless otherwise defined, all terms including technical and scientific terms used herein have the same meaning as commonly understood by those skilled in the technical field to which the present disclosure pertains. It will be understood that terms, such as those defined in commonly used dictionaries, should be interpreted as having a meaning that is consistent with their meaning in the context of the relevant art and will not be interpreted in an idealized or overly formal sense unless expressly so defined herein.

In addition, in the description with reference to the accompanying drawings, the same reference numerals are assigned to the same components regardless of the reference numerals, and redundant descriptions thereof will be omitted. In describing the embodiments, when it is determined that a detailed description of related known technologies may unnecessarily obscure the gist of the embodiments, the detailed description thereof will be omitted.

Components included in an embodiment and components having a common function will be described using the same names in other embodiments. Unless otherwise stated, descriptions described in an embodiment may be applied to other embodiments as well, and detailed descriptions within the overlapping range will be omitted.

Hereinafter, the composition for cancer diagnosis targeting cancer and/or cancer microenvironment and a use thereof of an embodiment of the present disclosure will be described in detail with reference to examples and drawings. However, the present disclosure is not limited to these examples and drawings.

An embodiment of the present disclosure provides a compound represented by the following [Formula 1] or a pharmaceutically acceptable salt thereof: 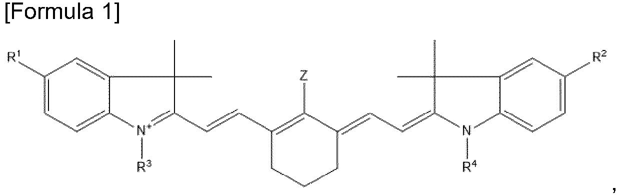
in which R¹ and R² are the same as or different from each other, and are each independently any one or more selected from the group consisting of hydrogen, a hydroxy group, a C₁-C₅ alkoxy group, (Y)ₘX, (Y)ₘCOO⁻, (Y)ₘSO₃⁻, (Y)ₘPO₃H⁻, and combinations thereof;
R³ and R⁴ are the same as or different from each other, and are each independently one or more selected from the group consisting of (Y)ₙH, (Y)ₙ(CO)R⁵, and combinations thereof;
R⁵ is a hydroxy group or 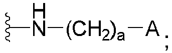
A is a C₂-C₆ heterocycloalkyl group containing any one or more heteroatoms selected from the group consisting of substituted or unsubstituted N, O, and S;
X is a halogen group,
Y is CH₂ or CH₂CH₂O;
Z is halogen or 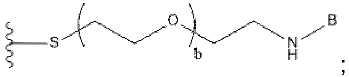
B is hydrogen or 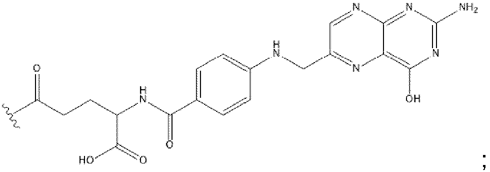 and
a, b, m, and n may be the same as or different from each other, and may each independently be an integer from 0 to 5.

As used herein, the term "substitution" refers to a reaction in which atoms or atom groups included in the molecule of the compound are replaced with other atoms or atom groups.

As used herein, the term "chain alkyl group" refers to a group derived from a straight-chain or branched-chain saturated aliphatic hydrocarbon having a specified number of carbon atoms and at least one valency. Examples of such alkyl groups include, but are not limited to, methyl, ethyl, propyl, isopropyl, butyl, isobutyl, 2-butyl, 3-butyl, pentyl, n-hexyl, and the like.

As used herein, the term "halogen group" refers to elements belonging to Group 17 of the periodic table and may be fluorine (F), chloride (Cl), bromine (Br), iodine (I), or the like.

As used herein, the term "alkoxy group" refers to an atomic group CₙH₂ₙ+O-formed by bonding an oxygen atom to an alkyl group. Examples of such alkoxy groups include, but are not limited to, methoxy, ethoxy, propoxy, butoxy, or the like.

As used herein, the term "heterocycloalkyl" refers to a stable 3- to 18-membered saturated or partially unsaturated radical which consists of 2 to 20 carbon atoms, desirably 2 to 15 carbon atoms, desirably 2 to 10 carbon atoms, and desirably 2 to 6 carbon atoms, and 1 to 6 heteroatoms, for example, 1 to 5 heteroatoms, 1 to 4 heteroatoms, 1 to 3 heteroatoms, or 1 or 2 heteroatoms selected from the group consisting of nitrogen, oxygen, and sulfur.

As used herein, the term "pharmaceutically acceptable salt" refers to a formulation of a compound that does not cause significant irritation to an organism to which it is administered and does not damage the biological activity and properties of the compound. The pharmaceutically acceptable salt may be obtained by allowing the compound of an embodiment of the present disclosure to react with inorganic acids such as hydrochloric acid, bromic acid, sulfuric acid, nitric acid, and phosphoric acid; sulfonic acids such as methanesulfonic acid, ethanesulfonic acid, and p-toluenesulfonic acid; or organic carbonic acids such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid and salicylic acid. In addition, the salts may be obtained by allowing the compound of an embodiment of the present disclosure to react with bases to form alkali metal salts such as ammonium salt, sodium salt and potassium salt; alkali earth metal salts such as calcium salt and magnesium salt; salts of organic bases such as dicyclohexylamine, N-methyl-D-glucamine and tris(hydroxymethyl)methylamine; and amino acid salts such as arginine and lysine.

In addition, an embodiment of the present disclosure may provide a contrast agent composition for cancer diagnosis including the compound or a pharmaceutically acceptable salt thereof as an active ingredient.

As used herein, the term "including...as an active ingredient" refers to the presence of the corresponding ingredient in an amount necessary or sufficient to achieve a desired biological effect. In real applications, the amount to be included as an active ingredient may be determined by considering that it is an amount for treating or diagnosing the target disease and does not cause other toxicity. For example, the amount of the active ingredient may vary depending on various factors, such as the disease or condition to be treated, the dosage form of a composition, the size of a subject or the severity of the disease or condition. The effective amount of the individual composition may be empirically determined by those skilled in the art without excessive experiments.

As used herein, the term "contrast agent" refers to a material administered to a body to potently and specifically contrast or image cancer cells *in vivo*, and is now widely used for image enhancement of tissues and cells in medical and diagnostic fields. The term "contrast agent" used herein is not limited to the range of Magnetic Resonance Imaging (MRI), Computed Tomography (CT), and Positron Emission Tomography (PET) contrast agents, and is used to include contrast agents for ultrasound imaging analysis and fluorescent imaging analysis.

In an embodiment of the present disclosure, the contrast agent composition may target a tumor and/or tumor microenvironment. The target may be included in the composition of a ligand that binds to a biomolecule overexpressed in the tumor and/or tumor microenvironment.

The term "overexpressed biomolecule" may be a receptor, biomarker, exosome, protein, genetic material, or enzyme that is expressed more highly in cancer cells and cancer microenvironment than in normal cells, desirably a receptor, and most desirably a folate receptor and a mannose receptor.

As used herein, the term "ligand" refers to a substance that forms a complex by binding to biomolecules overexpressed in a cancer and/or cancer microenvironment, specifically proteins, peptides, polypeptides, saccharides, organic acids, fatty acids, and glycoproteins, desirably a substrate that binds to a receptor, and most desirably folic acid or mannose.

The ligand may desirably be independently substituted at each of a -NH2 terminus and -COOH terminus of the compound of [Formula 1-3]. According to the most preferred embodiment of the present disclosure, folic acid may be substituted at a -NH2 terminus, and mannose may be substituted at a -COOH terminus.

According to one aspect, the -NH2 terminus may be modified with a ligand that binds to folate receptor alpha (FR α) in cancer cells and/or folate receptor beta (FR β) in tumor-associated macrophages (TAMs).

According to one aspect, the ligand is folic acid and may be represented by [Formula 1-4] below:

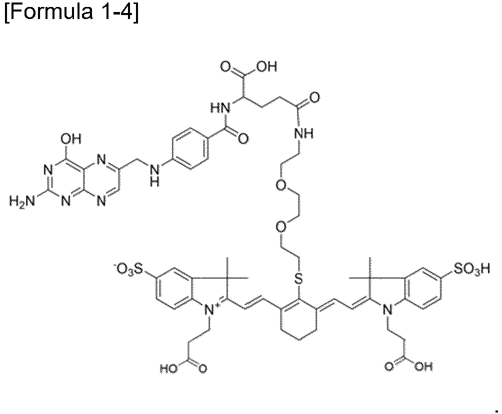

Folic acid is an essential nutrient for the division and growth of cancer cells and normal cells, and is absorbed into cells and metabolized through folate receptors. Folate receptors are overexpressed in various types of cancer cells, such as kidney cancer, ovarian cancer, pituitary tumor, and colon cancer, and may be used as a molecular target for diagnosis and treatment. The folic acid may specifically bind to the folate receptor overexpressed on the surface of the targeted cancer cells and inflammatory cells, thereby acting as a targeting ligand that allows the folic acid conjugate to be taken up into the targeted cells. More specifically, the folic acid may act by targeting overexpressed folate receptor alpha in cancer cells and folate receptor beta overexpressed in tumor-associated macrophages. Based on the properties of the compound of an embodiment of the present disclosure, the folic acid may have the benefit of simultaneously targeting cancer cells and tumor-associated macrophages.

In another embodiment of the present disclosure, the contrast agent composition may bind to any one or more of folate receptor alpha of cancer cells, folate receptor beta of tumor-associated macrophages (TAMs) in the tumor microenvironment, and CD206 of TAM.

The folate receptor functions to reduce folate derivatives by binding to folic acid, and is a receptor that mediates the delivery of folic acid into cells after binding to folic acid or folate derivatives. The folate receptor may be expressed to a limited extent in normal tissues, but is overexpressed in tumors and may be used as a target to selectively deliver drugs to tumor tissues.

Folate receptors exist in three isomeric forms: folate receptor alpha, beta, and gamma, of which alpha and beta are typically bound to the membrane of cells by a glycosylphosphatidylinositol (GPI) anchor, and may recycle between extracellular and endocytic compartments and transport folic acid intracellularly.

The folate receptor beta (FR β. FR-beta, FR B, FOLR-2 or FOLR2) is overexpressed in M2-type-polarized tumor-associated macrophages and may target tumor-associated macrophages within the cancer microenvironment. The folate receptor beta binds to folic acid and reduced folate derivatives and may mediate the transport of 5-methyl tetrahydrofolate and folate analogs into the cells, and has high affinity for folic acid and folate analogs at neutral pH. In addition, the exposure to slightly acidic pH after receptor endocytosis may significantly reduce the affinity for folic acid and induce conformational changes that mediate release.

The tumor-associated macrophages (TAMs) may desirably have the phenotype of an M2-type macrophage and exist in the cancer microenvironment, but are not particularly limited to the M2-type. The macrophage is one of the terminally differentiated phenotypes of myeloid cells, meaning that myeloid cells mainly existing in the bone marrow migrate to the cancer microenvironment through the blood and differentiate, and may be polarized into M1-macrophages and M2-macrophages under various physiological and pathological conditions *in vivo*. The tumor-associated macrophages play an important role in linking inflammation and cancer, promote tumor angiogenesis by increasing proliferation, invasion, and metastasis of cancer cells, and induce tumor progression by inhibiting anti-cancer immune responses mediated by T cells. The tumor-associated macrophages may be used as a potential treatment target for cancer and as a biomarker for diagnosis and prognosis of cancer.

The tumor microenvironment (TME) refers to the surrounding environment of a tumor or cancer, and is a totality composed of complex and diverse elements such as other cells around cancer cells, extracellular matrix, growth hormones, and signaling substances. The cancer microenvironment is composed of various immune cells regardless of the type or mutation of the cancer and is known to maintain a relatively similar environment.

According to one aspect, the -COOH terminus of the compounds of [Formula 1-3] and [Formula 1-4] may be modified into a ligand targeting cancer cells or a tumor-associated macrophage (TAM) receptor substrate.

The tumor-associated macrophage receptor may enable a cancer diagnostic composition to bind more specifically to M2-type macrophage tumor-associated macrophages.

The tumor-associated macrophage (TAM) receptor substrate may specifically bind to the tumor-associated macrophage (TAM) receptor and effectively target the tumor-associated macrophages (TAMs).

According to one aspect, the tumor-associated macrophage (TAM) receptor may desirably be CD206. The CD206 is a mannose receptor, which refers to a type I transmembrane protein with an extracellular N-terminus and an intracellular C-terminus. When applied to a contrast agent containing a fluorescent substance, the CD206 may express tumor-associated macrophages (TAMs) polarized into the M2-type and folate receptor beta.

According to one aspect, the tumor-associated macrophage (TAM) receptor substrate may be sugar, desirably mannose. The mannose is a receptor substrate that specifically binds to CD206, a tumor-associated macrophage receptor in the tumor microenvironment, and the compound modified as above may be represented by [Formula 1-5] or [Formula 1-6] below: 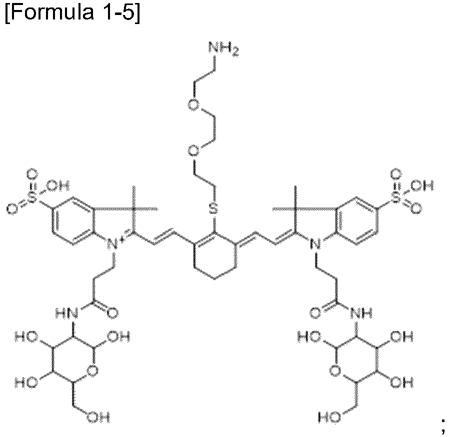 and

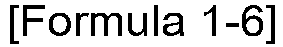

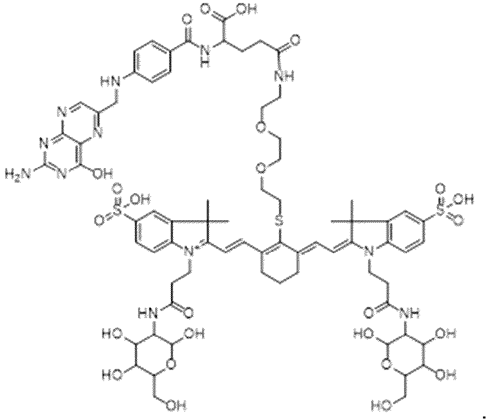

In another embodiment of the present disclosure, the contrast agent composition may simultaneously target the tumor and the tumor microenvironment.

In addition, an embodiment of the present disclosure provides a method for cancer diagnosis including targeting a tumor and/or tumor microenvironment using any one of the compositions.

Another aspect of the present disclosure provides a cancer diagnosis method that simultaneously targets folate receptor alpha-expressing cancer and the tumor microenvironment.

According to one embodiment, the folate receptor alpha-expressing cancer may be any one selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer, and medullary thyroid cancer.

The folate receptor alpha targets cancerous tumor cells, and the folate receptor beta targets tumor-associated macrophages.

As used herein, the term "diagnosis" is meant to include determining the susceptibility of a subject to a specific disease or disorder, determining whether a subject currently has a specific disease or disorder, determining the prognosis of a subject suffering from a specific disease or disorder, or performing therametrics (for example, monitoring the status of a subject in order to provide information about therapeutic efficacy) or theranostics (for example, diagnosing a disease using a substance that targets the lesion and simultaneously treating the disease by delivering the drug only to the affected site). Specifically, as used herein, the term "diagnosis" means determining whether lung cancer cells have invaded the visceral pleura elastin layer in a subject.

As used herein, the term "subject" is not limited to any mammal such as livestock or human that requires diagnosis, but may desirably be a human.

The contrast agent composition according to an embodiment of the present disclosure may be administered through various routes, including orally, transdermally, subcutaneously, intravenously, or intramuscularly, and the dosage of an active ingredient may be appropriately selected depending on various factors such as the route of administration, the patient's age, gender, weight, and patient's severity. In addition, the composition of an embodiment of the present disclosure may be administered in combination with known compounds that may enhance the desired effect.

In addition, an embodiment of the present disclosure provides an imaging method including treating the contrast agent composition in a biological sample. In addition, an embodiment of the present disclosure provides an imaging method including administering the contrast agent composition to a subject.

As used herein, the term "imaging" refers to all methods of visualizing a target object. As used herein, the term "imaging" may desirably be optical imaging using light.

The term "imaging" may be any one or more selected from the group consisting of fluorescence, bioluminescence, magnetic resonance imaging (MRI), computed tomography (CT), positron emission tomography (PET), single photon emission computed tomography (SPECT), and combinations thereof, but is not limited thereto.

The compounds of [Formula 1-1] to [Formula 1-6] of an embodiment of the present disclosure may function as fluorescent substances in a cancer environment. The fluorescent material is a material that develops color in response to light of a specific wavelength, and includes molecules, metal ions, complex compounds, organic dyes, conductors, semiconductors, insulators, and quantum dots that emit light in an excited state. Examples thereof include cyanine series, pyrene series, NIR series, Alexa series, and ZW series, but are not particularly limited.

The fluorescent material desirably refers to a contrast agent used in a fluorescence imaging method, which is one of the imaging methods used in cancer diagnosis, and uses a material that emits fluorescence when exposed to exited site light with a specific wavelength. In this connection, the body is exposed to exited site light outside the body, and fluorescence emitted from the fluorescent contrast agent inside the body is sensed.

The composition for cancer imaging may be a fluorescent contrast agent, and the fluorescent material used in the fluorescent contrast agent may desirably be a material that exhibits absorption in the near-infrared light region, 700 to 1300 nm, and may most desirably be excited at a wavelength of 779 nm and emit a wavelength of 807 nm. When using fluorescent substances in the visible light range corresponding to 400 to 600 nm, which is outside the wavelength range, the light transmittance through biological tissue is very low, and it is almost impossible to search for lesions deep in the body, while near-infrared light has high penetrability through biological tissue and may penetrate up to a skull size of approximately 10 cm.

The fluorescent wavelength range of the compound of an embodiment of the present disclosure is similar to that of Indocyanine Green (ICG), a fluorescent substance used in conventional cancer surgery, and thus may use fluorescence imaging devices currently used in clinical practices.

The fluorescent contrast agent may further include a pharmaceutically acceptable carrier. Pharmaceutically acceptable carriers are those commonly used in preparation, such as lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, but are not limited thereto.

### MODE FOR CARRYING OUT THE INVENTION

Hereinafter, the present disclosure will be described in detail with reference to the following examples and comparative examples. However, the technical idea of the present disclosure is not limited or restricted thereby.

### Preparation Example 1. Synthesis of Precursor 1

All chemicals and solvents used American Chemical Society or HPLC purity. HPLC grade methanol (MeOH), ethanol (EtOH), and distilled water (DW) were purchased from Fisher Scientific (Pittsburgh, PA, USA). All other chemicals, including dimethyl sulfoxide (DMSO), dimethylformamide (DMF), ethyl acetate (EA), and N,N-diisopropylethylamine (DIEA), were purchased from Fisher Scientific (Pittsburgh, PA, USA) and Sigma-Aldrich (St. Louis, MO, USA). The purity of all compounds was determined using a liquid chromatography-mass spectrometer (LC-MS) consisting of an Alliance e2695 separation module (Waters), a 2998PDA detector (Waters, 212-800 nm) and an Acquity QDA detector (Waters, m/z range: 50- 1,239). An XBridge C18 (4.6'150 mm, 5 µm) reverse-phase HPLC column (Waters) was used for LC-MS. The final compound was purified using preparative HPLC consisting of a Waters 2489 UV/Visible detector and a Waters 1525 Binary HPLC pump. An XBridge Prep C18 (19'150 mm, 5 µm) reverse-phase HPLC column (Waters) was used, and the purity of the final compound measured using a photodiode array (PDA) at 254/750 nm absorbance was 90% or higher.

### [Precursor 1]

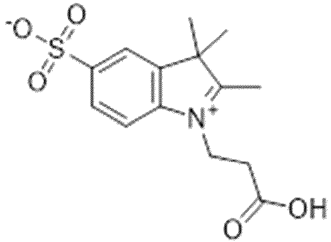

Precursor 1 (1-(2-carboxyethyl)-2,3,3-trimethyl-3H-indol-1-ium-5-sulfonate), (SC-COOH)) was synthesized as follows.

A mixture of potassium 2,3,3-trimethyl-3H-indole-5-sulfonate (10.0 g, 36 mmol) and 3-bromopropanoic acid (6.3 g, 42 mmol) in toluene (150 mL) solvent was heated in a nitrogen environment at 110°C for 24 hours. The mixture was cooled to room temperature and the solvent was decanted. Methanol (20 mL) was added to the mixture and stirred for 30 minutes to dissolve. The mixture was filtered and collected. Undissolved solids were removed by filtration. Then, the mixed solution was slowly added to ethyl acetate (700 mL) using a dropping funnel. The precipitate was filtered, dried and identified by LC-MS. This product was collected as a pink solid and used in the next stage without further purification (3.9 g, 35% yield). The ¹H NMR data and ¹³C NMR data of the product are as follows. Accurate mass TOF MS m/z [M]+ calculated for [C14H17NO5S]+ 311.08, found [M+H]+ 312.08. ¹H NMR (500 MHz, DMSO-d6) δ 8.01 - 7.90 (m, 2H), 4.74 (t, J = 8.6 Hz, 1H), 2.91 (d, J = 8.7 Hz, 1H), 1.71 (s, 3H). ¹³C NMR (125 MHz, DMSO-d6) δ 193.78, 175.40, 150.07, 140.22, 139.61, 130.80, 123.47, 117.50, 45.10, 44.17, 35.80, 26.13, 15.51.

### Preparation Example 2. Synthesis of Compound 1 (KBSF2)

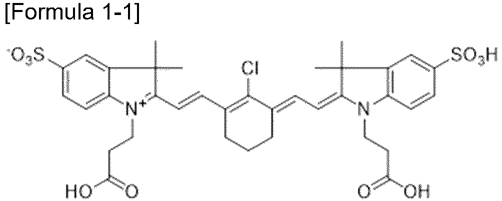

Compound 1 (3-(2-((E)-2-((E)-3-(2-((E)-1-(2-carboxyethyl)-3,3-dimethyl-5-sulfoindolin-2-ylidene)ethylidene)-2-chlorocyclohex-1-en-1-yl)vinyl)-3,3-dimethyl-5-sulfo-3H-indol-1-ium-1-yl)propanoate) was synthesized as follows.

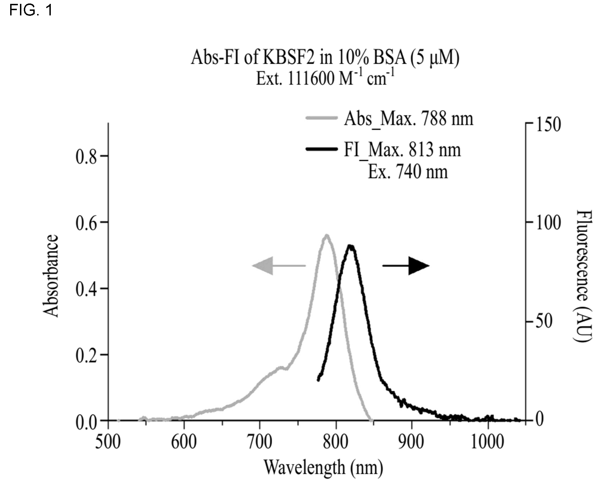

A mixture of bromide salt, precursor 1 (5.0 g, 16.1 mmol), Vilsmeier-Haack reagent (2.5 g, 7.1 mmol) and anhydrous sodium acetate (2.0 g, 24.1 mmol) in anhydrous ethanol (50 mL) solvent was heated under reflux at 85°C and stirred for 2 hours in a nitrogen environment. To obtain the best purity and yield, the molar ratio of Precursor 1 to Vilsmeier-Haack reagent was set higher than 2 : 1. To identify the completion of the reaction, the mixture was checked 2.5 hours after the reaction. After the reaction, the mixture was cooled to room temperature, filtered, washed with ethanol, collected, and dried to obtain Compound 1 as a dark green solid (4.4 g, 81% yield). The absorbance and fluorescence analysis results of Compound 1 are shown in FIG. 1. The ¹H NMR data and ¹³C NMR data of the prepared product are as follows. Accurate mass TOF MS m/z [M]+ calculated for [C36H39ClN2O10S2]+, found [M+H]+ 759.18. ¹H NMR (500 MHz, DMSO-d6) δ 8.09 - 8.02 (m, 2H), 7.97 (dd, J = 8.1, 2.2 Hz, 1H), 7.92 (d, J = 2.2 Hz, 1H), 7.82 (s, 1H), 7.65 (dd, J = 8.0, 2.1 Hz, 1H), 7.06 - 6.97 (m, 2H), 6.91 (dt, J = 15.1, 0.8 Hz, 1H), 6.69 - 6.58 (m, 2H), 4.84 (t, J = 8.5 Hz, 2H), 3.95 (t, J = 5.7 Hz, 2H), 2.88 (d, J = 8.6 Hz, 1H), 2.76 - 2.59 (m, 5H), 1.75 (s, 4H), 1.67 - 1.58 (m, 2H), 1.62 (s, 5H). ¹³C NMR (125 MHz, DMSO-d6) δ 176.20, 174.84, 171.84, 159.01, 148.89, 148.24, 144.16, 140.65, 139.47, 138.14, 136.77, 136.33, 135.09, 132.79, 131.26, 130.75, 129.06, 122.91, 122.63, 122.01, 121.31, 112.39, 96.28, 45.51, 45.19, 43.48, 43.35, 35.75, 32.46, 28.09, 28.06, 27.19, 27.09, 23.02.

### Preparation Example 3. Synthesis of Compound 2 (MAN-KBSF2)

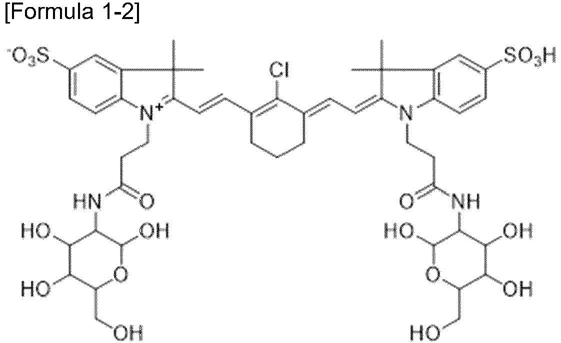

Compound 2 (2-((E)-2-((E)-2-chloro-3-(2-((E)-3,3-dimethyl-1-(3-oxo-3-(((3R,4R,5R,6S)-3,4,5-trihydroxy-6-(hydroxymethyl)tetrahydro-2H-pyran-2-yl)amino)propyl)-5-sulfoindolin-2-ylidene)ethylidene)cyclohex-1-en-1-yl)vinyl)-3,3-dimethyl-1-(3-oxo-3-(((3S,4S,5S,6R)-3,4,5-trihydroxy-6-(hydroxymethyl)tetrahydro-2H-pyran-2-yl)amino)propyl)-5-sulfo-3H-indol-1-ium) was synthesized as follows.

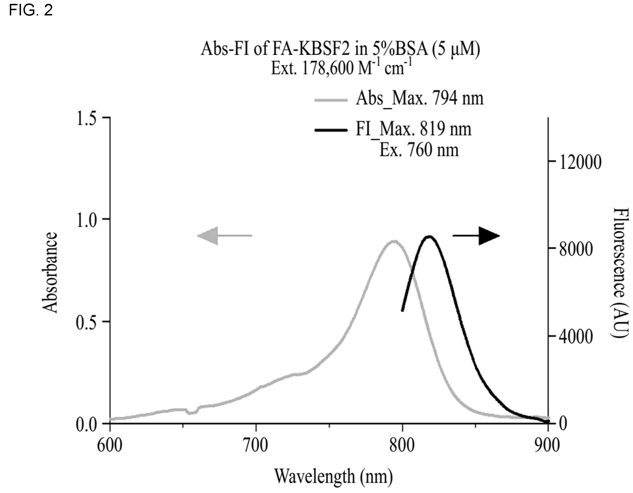

A mixture of Compound 1 (1.0 g, 1.32 mmol), (3R,4R,5R,6S)-2-amino-6-(hydroxymethyl)tetrahydro-2H-pyran-3,4,5-triol (0.6 g, 3.30 mmol), and DMTMM (1.83 g, 6.6 mmol) was sonicated in anhydrous methanol solvent and reacted for 6 hours in a nitrogen environment. After the reaction, the mixture was precipitated in EA, filtered, washed with EtOH, collected and dried to obtain Compound 2 as a dark green solid (0.7 g, 48% yield). The absorbance and fluorescence analysis results of Compound 2 are shown in FIG. 2. The ¹H NMR data and ¹³C NMR data of the prepared product are as follows. Accurate mass TOF MS m/z [M]+ calculated for [C48H61ClN4O18S2]+, found [M+H]+ 1081.31. ¹H NMR (500 MHz, DMSO-d6) δ 9.22 (d, J = 8.2 Hz, 1H), 8.09 - 8.02 (m, 1H), 8.00 - 7.90 (m, 1H), 7.83 - 7.75 (m, 1H), 7.65 (dd, J = 8.0, 2.1 Hz, 1H), 7.06 - 6.97 (m, 1H), 6.91 (dt, J = 15.1, 0.9 Hz, 1H), 6.69 - 6.58 (m, 1H), 5.70 (d, J = 3.2 Hz, 1H), 4.94 (td, J = 4.4, 3.2 Hz, 1H), 4.85 - 4.76 (m, 1H), 4.66 (d, J = 4.7 Hz, 1H), 4.48 - 4.40 (m, 2H), 3.91 (t, J = 5.1 Hz, 1H), 3.81 - 3.68 (m, 2H), 3.63 (ddd, J = 12.1, 4.5, 3.2 Hz, 1H), 3.48 (tt, J = 8.1, 4.7 Hz, 1H), 3.42 - 3.36 (m, 1H), 3.11 (td, J = 7.2, 4.6 Hz, 1H), 2.82 - 2.65 (m, 3H), 2.60 - 2.48 (m, 1H), 1.75 (s, 2H), 1.67 - 1.58 (m, 1H), 1.62 (s, 3H). ¹³C NMR (125 MHz, DMSO-d6) δ 173.00, 172.72, 171.84, 159.06, 148.89, 148.50, 143.38, 140.65, 139.60, 138.14, 136.77, 136.33, 135.09, 132.79, 131.26, 130.75, 129.06, 122.91, 122.63, 122.02, 121.24, 112.37, 96.28, 94.53, 94.48, 75.40, 72.12, 72.09, 70.68, 61.63, 55.75, 55.72, 45.50, 45.33, 44.21, 43.35, 34.92, 34.36, 28.09, 28.06, 27.19, 27.08, 23.02.

### Preparation Example 4. Synthesis of Compound 3 (KBSF2-PEG-NH2)

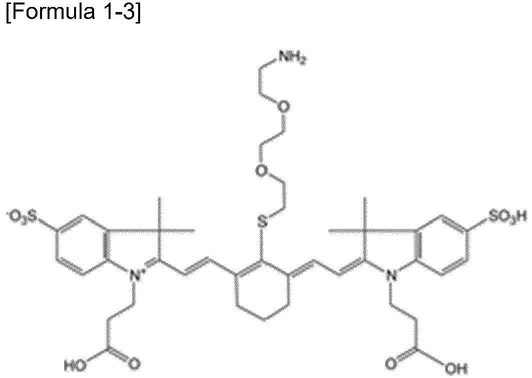

Compound 3 (2-((E)-2-((E)-2-((2-(2-(2-aminoethoxy)ethoxy)ethyl)thio)-3-(2-((E)-1-(2-carboxyethyl)-3,3-dimethyl-5-sulfoindolin-2-ylidene)ethylidene)cyclohex-1-en-1-yl)vinyl)-1-(2-carboxyethyl)-3,3-dimethyl-5-sulfo-3H-indol-1-ium) was synthesized as follows.

Compound 1 (1.0 g, 1.32 mmol) was added to 8 mL of distilled water in a 100 mL round bottom flask. DIEA (0.4 ml; 2.34 mmol; 2.0 molar equivalent) was pipetted into the flask and mixed, and tert-butyl (2-(2-(2-mercaptoethoxy)ethoxy)ethyl)carbamate (390 mg; 1.47 mmol; 1.11 molar equivalent) was added to 2mL of DMSO solvent and was added to the reaction solution heated in an oil bath at 60°C for 40 minutes. At 40 minutes, the reaction situation was identified using an LC-MS system. 1 mL of 6 M HCl was added at 60°C and the reaction was continued for 0.5 hours for deprotection. After the reaction, it was cooled to room temperature, slowly poured into 100 mL of acetone/EA (1 : 1) mixture, and stirred at room temperature for 30 minutes. The suspension was filtered and the precipitate was collected. The precipitate was dried at room temperature overnight and Compound 3 was collected as a dark green solid (0.9 g, 57% yield). The ¹H NMR data and ¹³C NMR data of the prepared product are as follows. Accurate mass TOF MS m/z [M]+ calculated for [C54H75N5O20S3]+, found [M+H]+ 1210.62. ¹H NMR (500 MHz, DMSO-d6) δ 8.09 - 8.02 (m, 1H), 8.00 - 7.90 (m, 1H), 7.83 - 7.75 (m, 1H), 6.27 - 6.20 (m, 1H), 5.70 (d, J = 3.2 Hz, 1H), 4.94 (td, J = 4.4, 3.2 Hz, 1H), 4.85 - 4.76 (m, 1H), 4.66 (d, J = 4.7 Hz, 1H), 4.48 - 4.40 (m, 2H), 3.91 (t, J = 5.1 Hz, 1H), 3.77 (dd, J = 8.4, 7.1, 4.5, 1.5 Hz, 1H), 3.76 - 3.67 (m, 2H), 3.63 (s, 1H), 3.67 - 3.59 (m, 1H), 3.53 - 3.36 (m, 2H), 3.15 - 3.02 (m, 2H), 2.86 - 2.74 (m, 1H), 2.77 - 2.70 (m, 2H), 2.70 (td, J = 7.1, 0.9 Hz, 1H), 2.60 - 2.48 (m, 1H), 1.75 (s, 1H), 1.62 (s, 2H), 1.64 - 1.56 (m, 1H). ¹³C NMR (125 MHz, DMSO-d6) δ 173.50, 172.88, 172.72, 160.01, 148.50, 145.14, 144.05, 143.38, 140.65, 140.57, 139.60, 138.14, 136.77, 134.54, 131.26, 130.31, 129.06, 122.91, 122.63, 121.24, 120.13, 112.37, 95.98, 94.53, 94.48, 75.40, 72.97, 72.12, 72.09, 71.01, 70.68, 69.99, 69.30, 61.63, 55.75, 55.72, 45.50, 45.33, 44.21, 43.35, 41.72, 34.92, 34.36, 31.78, 28.09, 27.76, 27.08, 26.62, 22.86.

### Preparation Example 5. Synthesis of Compound 4 (FA-KBSF2)

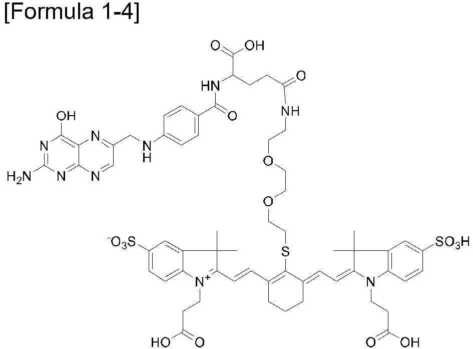

Compound 4 (2-((E)-2-((E)-2-((1-(4-(((2-amino-4-oxo-1,4-dihydropteridin-7-yl)methyl)amino)phenyl)-3-carboxy-1,6-dioxo-10,13-dioxa-2,7-diazapentadecan-15-yl)thio)-3-(2-((E)-1-(2-carboxyethyl)-3,3-dimethyl-5-sulfoindolin-2-ylidene)ethylidene)cyclohex-1-en-1-yl)vinyl)-1-(2-carboxyethyl)-3,3-dimethyl-5-sulfo-3H-indol-1-ium) was synthesized as follows.

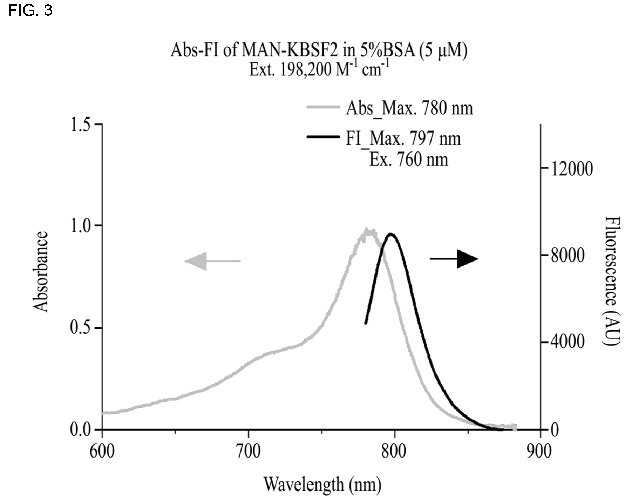

Folic acid (4 mg, 9.1 nmol), EDC (2 mg), and HOBt (1.4 mg) were added to 1 mL of DMSO in a 1.5 mL EP tube. DIEA (5 µl) was added to the reaction solution under vortexing for 2 hours at room temperature. 7.2 mg of Compound 3 (8.1 nm) was added and the reaction was continued for 4 hours. The suspension was filtered and the precipitate was collected. The precipitate was dried at room temperature overnight and Compound 4 was collected as a dark green solid (4.8 mg, 45% yield). The absorbance and fluorescence analysis results of Compound 4 are shown in FIG. 3. The ¹H NMR data and ¹³C NMR data of the prepared product are as follows. Accurate mass TOF MS m/z [M]+ calculated for [C61H70N10O17S3]+, found [M+H]/2+ 655.50. ¹H NMR (500 MHz, DMSO-d6) δ 8.60 (t, J = 0.8 Hz, 1H), 8.53 (d, J = 8.7 Hz, 1H), 8.09 - 8.02 (m, 2H), 7.97 (dd, J = 8.1, 2.2 Hz, 1H), 7.92 (d, J = 2.2 Hz, 1H), 7.83 - 7.75 (m, 2H), 7.75 - 7.70 (m, 2H), 7.65 (dd, J = 8.0, 2.1 Hz, 1H), 7.09 - 7.01 (m, 2H), 6.96 (d, J = 14.6 Hz, 1H), 6.85 - 6.79 (m, 2H), 6.63 - 6.58 (m, 3H), 6.27 - 6.20 (m, 2H), 4.84 (t, J = 8.5 Hz, 2H), 4.59 (dd, J = 6.0, 0.9 Hz, 2H), 4.32 (dt, J = 8.7, 6.5 Hz, 1H), 3.95 (t, J = 5.7 Hz, 2H), 3.70 (t, J = 5.5 Hz, 2H), 3.64 (s, 4H), 3.57 (t, J = 4.3 Hz, 2H), 3.42 (dt, J = 5.1, 4.3 Hz, 2H), 3.05 (d, J = 10.8 Hz, 1H), 2.89 (t, J = 8.5 Hz, 2H), 2.77 - 2.70 (m, 4H), 2.63 (d, J = 5.7 Hz, 1H), 2.28 (dt, J = 16.3, 8.7 Hz, 1H), 2.16 (dt, J = 16.4, 8.8 Hz, 1H), 2.03 (m, 1H), 1.81 (m, 1H), 1.75 (s, 3H), 1.62 (s, 5H), 1.64 - 1.56 (m, 2H). ¹³C NMR (125 MHz, DMSO-d6) δ 176.20, 174.84, 174.54, 174.03, 172.88, 167.01, 163.86, 159.92, 156.75, 154.64, 150.23, 148.24, 146.50, 146.31, 145.14, 144.16, 144.05, 140.65, 140.57, 139.47, 138.14, 136.77, 134.54, 131.26, 130.42, 130.31, 129.90, 129.06, 124.74, 122.91, 122.63, 121.31, 120.14, 112.55, 112.39, 95.98, 72.95, 69.74, 69.34, 69.30, 53.15, 45.89, 45.51, 45.19, 43.48, 43.36, 40.06, 35.75, 32.46, 32.13, 31.78, 28.09, 27.76, 27.09, 26.62, 22.86.

### Preparation Example 6. Synthesis of Compound 5 (MAN-KBSF2-PEG-NH2)

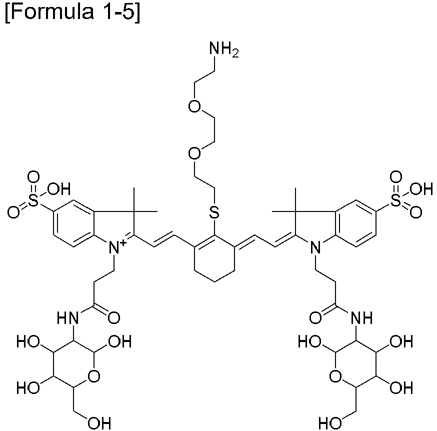

Compound 5 (2-((E)-2-((E)-2-((2-(2-(2-aminoethoxy)ethoxy)ethyl)thio)-3-(2-((E)-3,3-dimethyl-1-(3-oxo-3-((2,4,5-trihydroxy-6-(hydroxymethyl)tetrahydro-2H-pyran-3-yl)amino)propyl)-5-sulfoindolin-2-ylidene)ethylidene)cyclohex-1-en-1-yl)vinyl)-3,3-dimethyl-1-(3-oxo-3-((2,4,5-trihydroxy-6-(hydroxymethyl)tetrahydro-2H-pyran-3-yl)amino)propyl)-5-sulfo-3H-indol-1-ium) was synthesized as follows.

Compound 2 (MAN-KBSF2; 1.0 g, 0.92 mmol) was dissolved in 8 mL of distilled water and added to a 100 mL round bottom flask. DIEA (0.4 ml; 2.34 mmol; 2.5 molar equivalent) was added to the flask and mixed well, and Tert-butyl(2-(2-(2-mercaptoethoxy)ethoxy)ethyl)carbamate (270 mg; 1.02 mmol; 1.11 molar equivalent) dissolved in 2 mL of DMSO was added to the reaction solution and heated in an oil bath at 60 degrees for 40 minutes. After 40 minutes, the progress was checked using the LC-MS system. 1.5 mL of 6 M HCl was added at 60 degrees and the reaction was continued for 30 minutes for deprotection. After the reaction, it was cooled to room temperature, slowly poured into 100 mL of acetone/EA (1 : 1) mixture, and stirred at room temperature for 30 minutes, and the suspension was filtered to collect the precipitate. The precipitate was dried at room temperature overnight and collected as a dark green solid (0.68 g, 61% yield). The ¹H NMR data and ¹³C NMR data of the prepared product are as follows. Accurate mass TOF MS m/z [M]+ calculated for [C54H75N5O20S3]+, found [M+H]/2+ 605.25. ¹H NMR (500 MHz, DMSO-d6) δ 8.16 - 8.07 (m, 1H), 7.83 - 7.69 (m, 1H), 6.27 - 6.20 (m, 1H), 5.70 (d, J = 3.2 Hz, 1H), 4.94 (td, J = 4.4, 3.2 Hz, 1H), 4.85 - 4.76 (m, 1H), 4.66 (d, J = 4.7 Hz, 1H), 4.48 - 4.40 (m, 2H), 3.91 (t, J = 5.1 Hz, 1H), 3.81 - 3.67 (m, 2H), 3.63 (s, 1H), 3.67 - 3.59 (m, 1H), 3.53 - 3.37 (m, 2H), 3.15 - 3.02 (m, 2H), 2.86 - 2.74 (m, 1H), 2.77 - 2.71 (m, 2H), 2.73 - 2.67 (m, 1H), 2.60 - 2.48 (m, 1H), 1.75 (s, 2H), 1.62 (s, 2H), 1.64 - 1.56 (m, 1H). ¹³C NMR (125 MHz, DMSO-d6) δ 173.00, 172.88, 172.72, 160.01, 148.79, 145.14, 144.05, 143.38, 140.69, 140.57, 138.94, 138.14, 136.77, 134.54, 132.45, 130.31, 129.06, 123.57, 122.68, 122.63, 120.14, 112.37, 95.98, 94.53, 94.48, 75.40, 72.97, 72.12, 72.09, 71.01, 70.68, 69.99, 69.30, 61.63, 55.75, 55.72, 45.50, 45.33, 44.21, 43.27, 41.72, 34.92, 34.36, 31.78, 28.09, 27.76, 27.09, 26.62, 22.84.

### Preparation Example 7. Synthesis of Compound 6 (FMK-2)

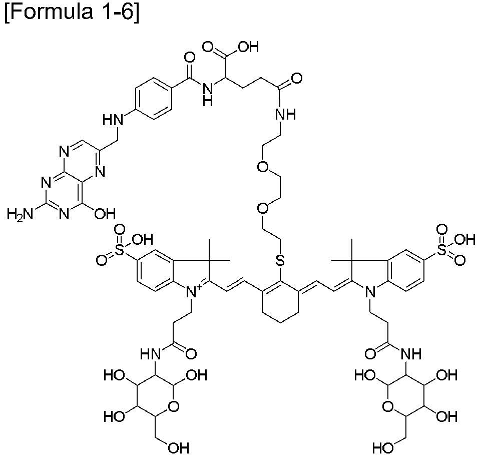

Compound 6 (2-((E)-2-((E)-2-((1-(4-(((2-amino-4-oxo-1,4-dihydropteridin-7-yl)methyl)amino)phenyl)-3-carboxy-1,6-dioxo-10,13-dioxa-2,7-diazapentadecan-15-yl)thio)-3-(2-((E)-3,3-dimethyl-1-(3-oxo-3-(((3R,4R,5R,6S)-3,4,5-trihydroxy-6-(hydroxymethyl)tetrahydro-2H-pyran-2-yl)amino)propyl)-5-sulfoindolin-2-ylidene)ethylidene)cyclohex-1-en-1-yl)vinyl)-3,3-dimethyl-1-(3-oxo-3-(((3S,4S,5S,6R)-3,4,5-trihydroxy-6-(hydroxymethyl)tetrahydro-2H-pyran-2-yl)amino)propyl)-5-sulfo-3H-indol-1-ium) was synthesized as follows.

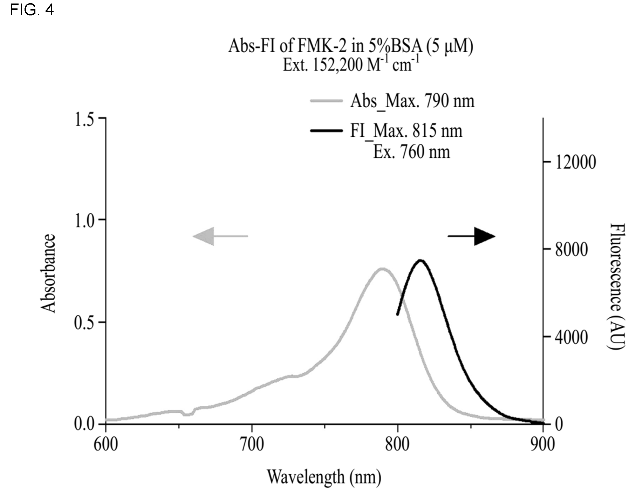

Folic acid (4 mg, 9.1 nmol), EDC (2 mg), and HOBt (1.4 mg) were added to 1 mL of DMSO in a 1.5 mL EP tube. DIEA (5 µl) was added to the reaction solution under vortex flow conditions for 2 hours at room temperature. 10 mg of Compound 5 (MAN-KBSF2-PEG-NH2 (8.3 nmol)) was added and the reaction was continued for 4 hours. The suspension was filtered to collect the precipitate and dried overnight at room temperature to collect a dark green solid (5.7 mg, 42% yield). The absorbance and fluorescence analysis results of Compound 6 are shown in FIG. 4. The ¹H NMR data and ¹³C NMR data of the prepared product are as follows. Accurate mass TOF MS m/z [M]+ calculated for [C61H70N10O17S3]+, found [M+H]/2+ 655.50. ¹H NMR (500 MHz, DMSO-d6) δ 8.60 (t, J = 0.8 Hz, 1H), 8.53 (d, J = 8.7 Hz, 1H), 8.09 - 8.02 (m, 2H), 7.97 (dd, J = 8.1, 2.2 Hz, 1H), 7.92 (d, J = 2.2 Hz, 1H), 7.83 - 7.75 (m, 2H), 7.75 - 7.70 (m, 2H), 7.65 (dd, J = 8.0, 2.1 Hz, 1H), 7.09 - 7.01 (m, 2H), 6.96 (d, J = 14.6 Hz, 1H), 6.85 - 6.79 (m, 2H), 6.63 - 6.58 (m, 3H), 6.27 - 6.20 (m, 2H), 4.84 (t, J = 8.5 Hz, 2H), 4.59 (dd, J = 6.0, 0.9 Hz, 2H), 4.32 (dt, J = 8.7, 6.5 Hz, 1H), 3.95 (t, J = 5.7 Hz, 2H), 3.70 (t, J = 5.5 Hz, 2H), 3.64 (s, 4H), 3.57 (t, J = 4.3 Hz, 2H), 3.42 (dt, J = 5.1, 4.3 Hz, 2H), 3.05 (d, J = 10.8 Hz, 1H), 2.89 (t, J = 8.5 Hz, 2H), 2.77 - 2.70 (m, 4H), 2.63 (d, J = 5.7 Hz, 1H), 2.28 (dt, J = 16.3, 8.7 Hz, 1H), 2.16 (dt, J = 16.4, 8.8 Hz, 1H), 2.03 (m, 1H), 1.81 (m, 1H), 1.75 (s, 3H), 1.62 (s, 5H), 1.64 - 1.56 (m, 2H). ¹³C NMR (125 MHz, DMSO-d6) δ 176.20, 174.84, 174.54, 174.03, 172.88, 167.01, 163.86, 159.92, 156.75, 154.64, 150.23, 148.24, 146.50, 146.31, 145.14, 144.16, 144.05, 140.65, 140.57, 139.47, 138.14, 136.77, 134.54, 131.26, 130.42, 130.31, 129.90, 129.06, 124.74, 122.91, 122.63, 121.31, 120.14, 112.55, 112.39, 95.98, 72.95, 69.74, 69.34, 69.30, 53.15, 45.89, 45.51, 45.19, 43.48, 43.36, 40.06, 35.75, 32.46, 32.13, 31.78, 28.09, 27.76, 27.09, 26.62, 22.86.

### Example 1. Fluorescent Contrast Observation

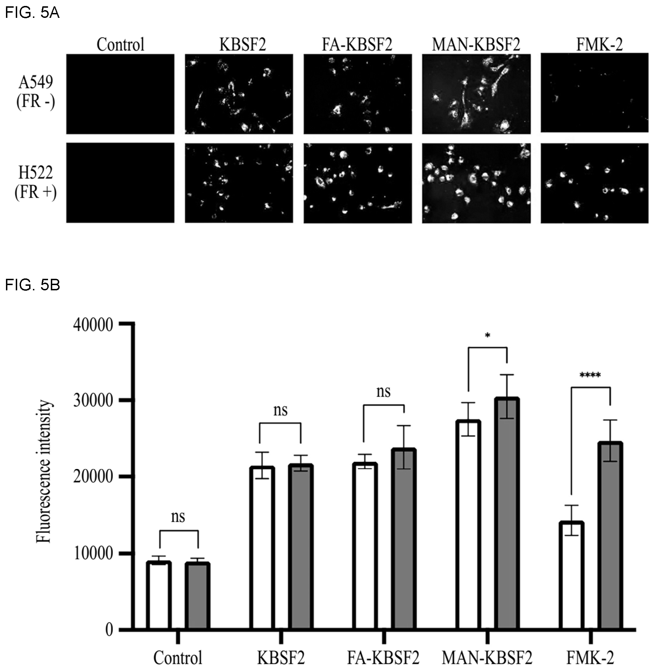

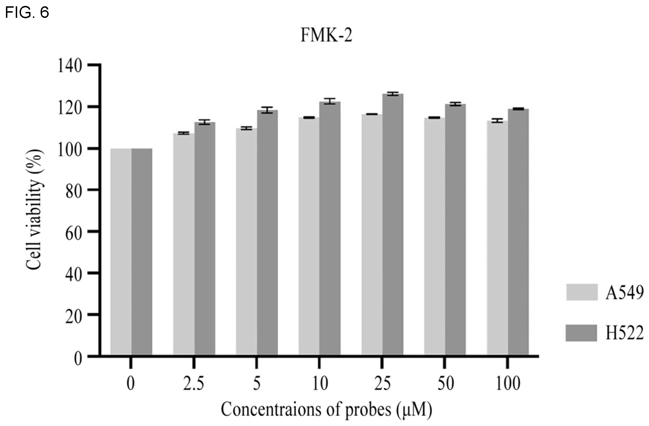

After preparing a fluorescent contrast agent using Compound 1 (KBSF2), Compound 2 (MAN-KBSF2), Compound 4 (FA-KBSF2), and Compound 6 (FMK-2) among the compounds prepared in the above preparation examples, A549 cancer cells, which do not express folate receptors, and H522 cancer cells, which express folate receptors, were treated respectively, and the results are shown in FIG. 5A and FIG. 5B. As shown in the figure, it was identified that Compounds 1, 2, and 4 of the present disclosure expressed fluorescence in cancer cells regardless of whether folate receptors were expressed, but Compound 6 (FMK-2) enabled fluorescence imaging specifically in cells expressing folate receptors. In particular, as shown in FIG. 6 below, it was identified that when Compound 6 (FMK-2) was treated at different concentrations to two lung cancer cell lines, A549 and H522, cell viability did not decrease. Accordingly, it was identified that FMK-2 had no cytotoxicity. It was assumed that the slightly increased cell viability compared to the untreated case was caused by this influence that FA (folic acid) promotes cell growth.

### Example 2. Mouse Fluorescence Experiment

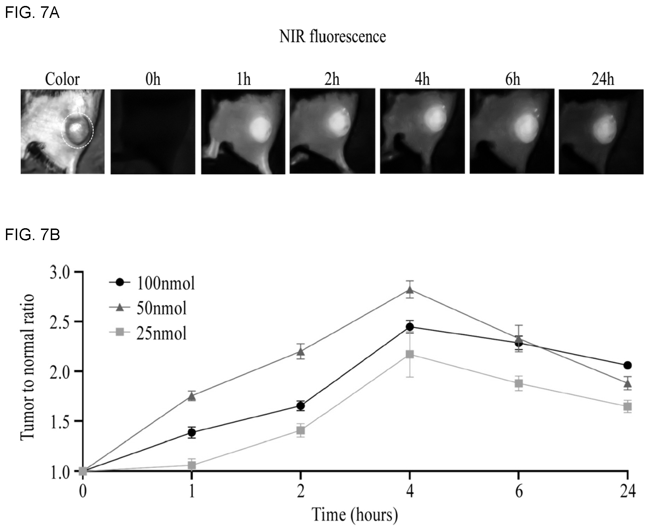

Compound 6 (FMK-2) was injected intravenously at 25 nmol, 50 nmol, and 100 nmol, respectively, into cancer-induced mice, and near-infrared fluorescence signals were observed for each time period, and the results are shown in FIG. 7A and FIG. 7B. As can be seen in the figure, a strong near-infrared fluorescence signal was able to be detected in cancer compared to normal tissue. In addition, at a concentration of 50 nmol, the ratio of fluorescence signal was highest compared to normal tissue, and the luminescence signal was measured the strongest at 4 hours after injection.

### Example 3. Immunochemical Analysis

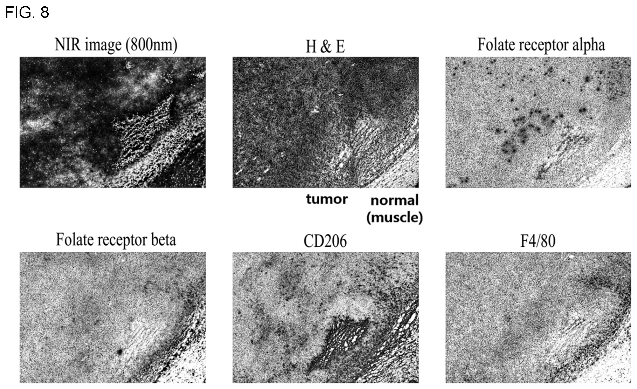

In Example 2, cancer tissue and surrounding normal tissue of mice injected intravenously with Compound 6 (FMK-2) were obtained, frozen, and sliced (cryosection). Near-infrared fluorescence signals were identified using a fluorescence microscope to identify the distribution of Compound 6, and immunohistochemistry analysis was performed along with H&E staining to identify the distribution of TAM (Tumor-associated macrophages). The results are shown in FIG. 8. In the H&E staining photo, the left side of the yellow dotted line shown as a diagonal line in the lower right corner was identified to be cancerous tissue, and the right side was identified to be normal tissue. Immunochemical staining results showed that folate receptor alpha, which is known to be overexpressed in cancer cells, was distributed in brown color in cancer tissue, and folate receptor beta, CD206, and F4/80, which are known as TAM markers, were all widely distributed at the border between cancer and normal tissue. In particular, it was identified that the fluorescence distribution in the near-infrared fluorescence image was similar to the area where folate receptor beta was mainly expressed. Accordingly, it was identified that Compound 6 (FMK-2) of the present disclosure was able to effectively target TAM and folate receptors, and that it was able to be used as a fluorescent contrast agent to distinguish cancer tissue from normal tissue.

### Example 4. Mouse Tissue Staining Experiment

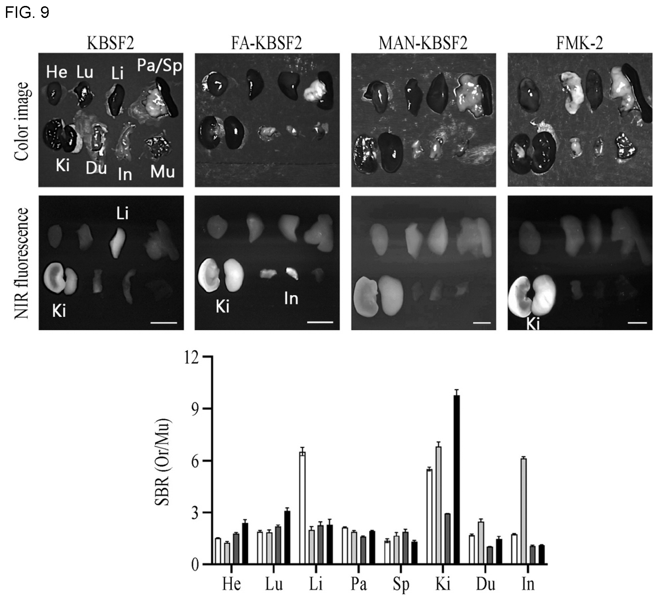

In order to identify in which organs each compound of the present disclosure is accumulated and excreted, each fluorescent substance was injected into a normal mouse, and each organ was extracted 4 hours later and fluorescence images were taken, and the results are shown in FIG. 9. Each graph represents the fluorescence signal of each organ. In the case of KBSF2 of the present disclosure, signals were higher in the liver and kidneys compared to other organs, signals were identified in the kidneys and small intestine for FA-KBSF2, and overall signals were identified in all organs for MAN-KBSF2. In particular, FMK-2 showed high signal only in the kidneys. Accordingly, it was identified that FMK-2 was excreted into the kidneys without non-specific binding to other organs.

### Example 5. Pharmacokinetic Analysis of Compound 6

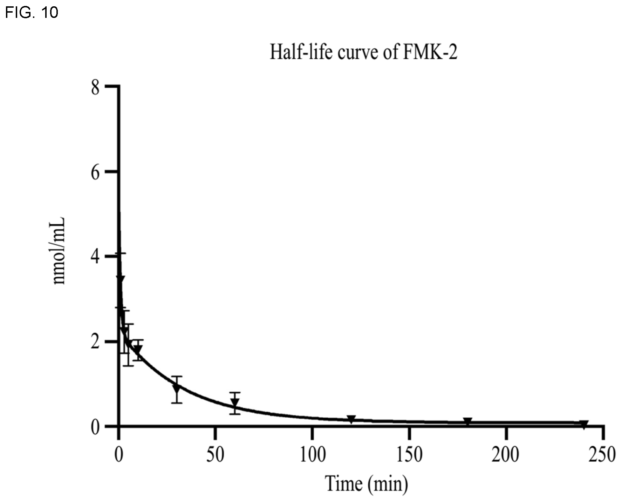

Pharmacokinetic parameter analysis was performed on Compound 6, and the results are shown in FIG. 10 and Table 1. The half-lives of distribution (T_{1/2α}) were measured at 1.1 minutes, and the half-lives of elimination phase (T_{1/2β}) were measured at 26.9 minutes. The area under the curve (AUC) value was measured at 100.6, and urine output 4 hours after intravenous injection of 100 nmol of FMK-2 was measured at 42%.

**[Table 1]**

| **Pharmacokinetic parameters** | |
|---|---|
| Molecular weight (g/mol) | 1634.79 |
| Injected dose (µmol/kg) | 3.0 |
| Cₘₐₓ (nmol/mL) | 69.46 |
| T_{1/2α} (min) | 1.11 |
| T_{1/2β} (min) | 26.90 |
| Urinary excretion (%ID), 4 h | 42 |
| AUC (min*nmol/mL) | 100.6 |
| Plasma clearance (mL/min) | 1.046 |
| Volume of distribution (mL) | 38.97 |

### Example 6. Comparative Experiment of Staining with Inflammatory Tissue

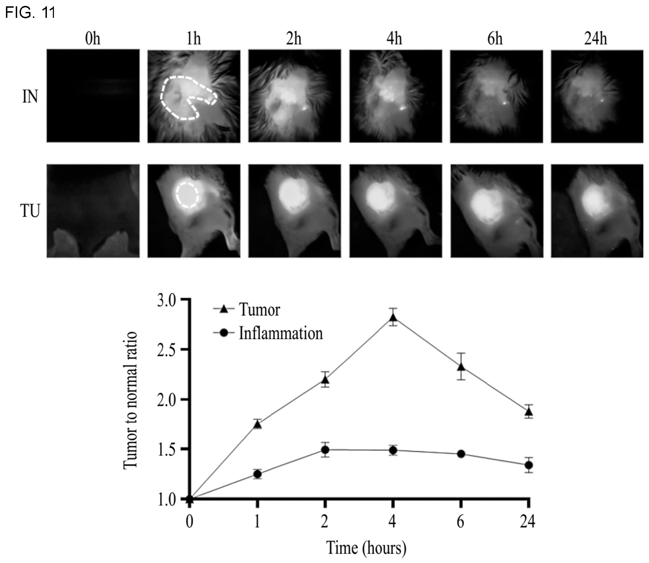

In order to verify how specifically Compound 6 of the present disclosure is able to target cancer tissue compared to inflammatory tissue, the following experiment was performed. Inflammation was induced in mice or a cancer model was established in mice, FMK-2 was injected intravenously in each of the mice, and fluorescence images were taken over time, and the results are shown in FIG. 11. In the case of the inflammation model, the signal weakened significantly over time and there was no specific binding to the inflammatory tissue compared to the normal tissue, whereas in the case of the cancer model, the signal was vividly visible even until 24 hours and the signal contrast between cancer and normal tissue was also strong. In the case of the graph, it shows the value of the fluorescence signal ratio of inflammatory or cancerous tissue compared to normal tissue. In cancer tissue staining, the highest fluorescence emission was identified at 4 hours, and the high level of fluorescence was identified to be maintained until 24 hours. In contrast, inflammatory tissue emitted a similar level of fluorescence for 2 to 24 hours, and the intensity was measured to be lower than that of cancer tissue.

### Comparative Example 1. Comparative Experiment with ZW800-PEG

In order to identify the difference in effect between ZW800-PEG (Non-Patent Document 1), conventionally known to have a zwitterionic near-infrared fluorophore, and Compound 1 (KBSF2) of the present disclosure, a comparative experiment was conducted as follows.

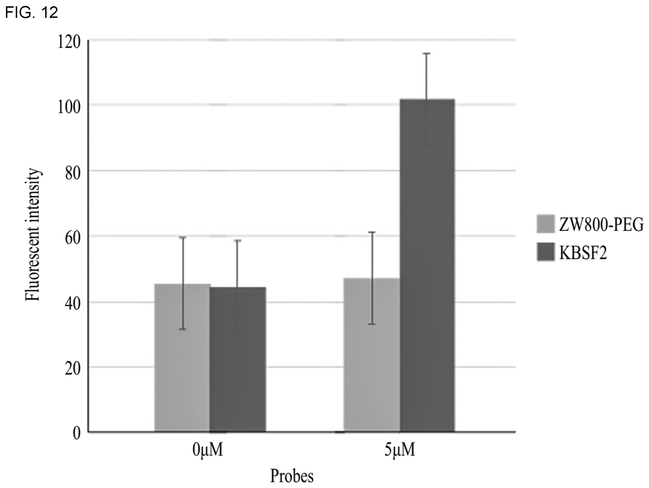

The experiment used human lung cancer cell line H522, which was purchased from ATCC (Manassas, VA). Cells were maintained in Roswell Park Memorial Institute (RPMI) 1640 medium supplemented with 10% fetal bovine serum (FBS) and 1% penicillin/streptomycin (p/s) at 37°C in a humidified 5% CO2 environment. To assess the binding affinity of the probe to cells, lung cancer cells were incubated at 37°C for 1 hour in Hank's Balanced Salt Solution (HBSS) of 10% FBS without phenol red without dye or in the presence of 5 µM ZW800-PEG and Compound 1 (KBSF2). After washing with HBSS without FBS, cells were imaged using a fluorescence microscope (EVOS FL Auto Imaging System; Life Technologies, Carlsbad, CA). All NIR fluorescence images for a particular fluorophore were normalized to be the same for all conditions during the experiment. In other words, brightness and contrast were optimized for the image with the strongest signal, and the same was applied to all other images acquired for that particular fluorophore. The results are shown in FIG. 12. As can be seen in the figure, the fluorescence intensity of KBSF2 of the present disclosure was identified to be more than twice as strong in the presence of 5 µM probe compared to ZW800-PEG, the related art, thus identifying that the fluorescence contrast effect in the near-infrared region was clearly superior when treated with 5 µM thereof.

### Comparative Example 2. Comparative Experiment with OCTL14

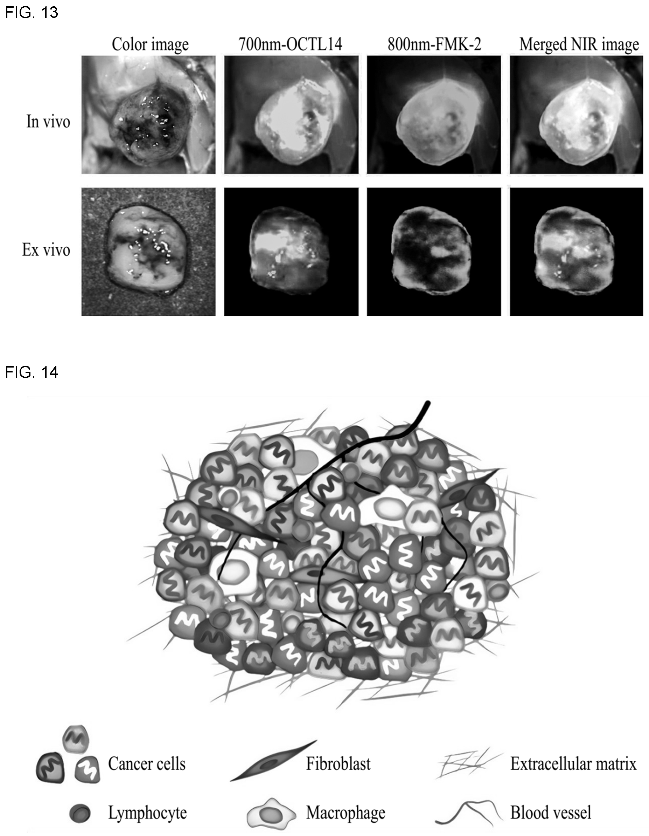

*In vivo* and *ex vivo* comparative experiments were conducted on OCTL14, a conventionally known contrast agent, and Compound 6 (FMK-2) of the present disclosure. After intravenously injecting OCTL14 and FMK-2 into the mouse cancer model with time difference, the cancer tissue was captured as fluorescence images after a certain period of time, which is shown in FIG. 13. In the case of OCTL14, it has a fluorescence wavelength of 700 nm, so fluorescence images were taken at the corresponding wavelength, and FMK-2 was captured as fluorescence images at 800 nm. OCTL14, which is known to accumulate in cancer tissue, was identified to accumulate inside the cancer tissue (tumor stroma), and FMK-2 was identified to accumulate in the tumor microenvironment (tumor nest).

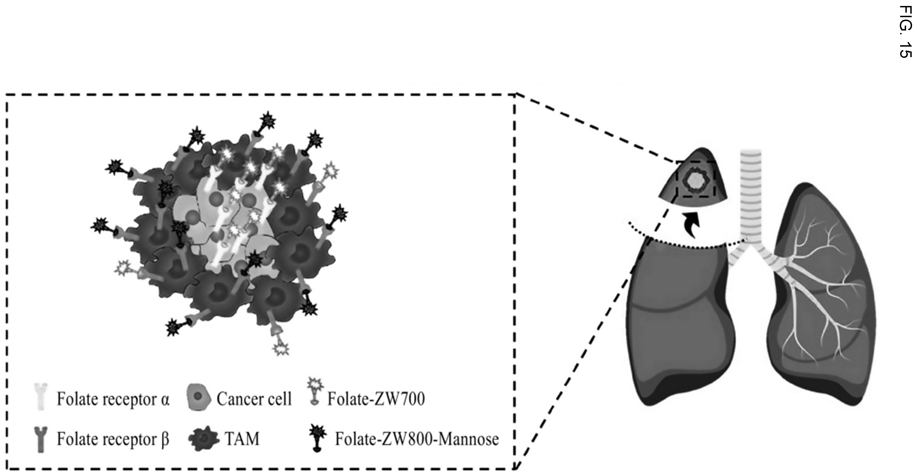

In addition, a schematic diagram of the tumor microenvironment, which is an example where the compound of the present disclosure and a contrast agent composition containing the same may be most appropriately applied, is shown in FIG. 14, and a schematic diagram of the fluorescence expression mechanism is shown in FIG. 15.

Although the embodiments have been described based on the limited embodiments and drawings as described above, those skilled in the pertinent technical field may apply various technical modifications and variations from the foregoing descriptions. For example, even when the described technologies are performed in a different order from that in the described method, and/or the described components such as a system, structure, device, and circuit are coupled or combined in a manner different from that as described above, or are replaced or substituted with other components or equivalents, appropriate results may be achieved.

Therefore, other implementations, other embodiments, and equivalents to claims also fall within the scope of the following claims.

## Claims

1. A compound represented by [Formula 1] below or a pharmaceutically acceptable salt thereof: 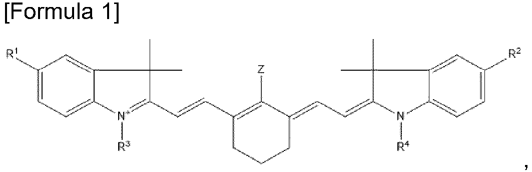
wherein R¹ and R² are the same as or different from each other, and are each independently any one or more selected from the group consisting of hydrogen, a hydroxy group, a C₁-C₅ alkoxy group, (Y)ₘX, (Y)ₘCOO⁻, (Y)ₘSO₃⁻, (Y)ₘPO₃H⁻, and combinations thereof;
R³ and R⁴ are the same as or different from each other, and are each independently any one or more selected from the group consisting of (Y)ₙH, (Y)ₙ(CO)R⁵, and combinations thereof;
R⁵ is a hydroxy group or 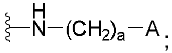
A is a C₂-C₆ heterocycloalkyl group containing any one or more heteroatoms selected from the group consisting of substituted or unsubstituted N, O, and S;
X is a halogen group,
Y is CH₂ or CH₂CH₂O;
Z is halogen or 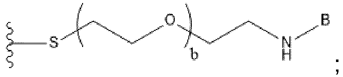
B is hydrogen or 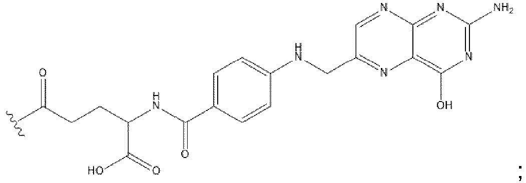 and
a, b, m, and n are the same as or different from each other, and are each independently an integer from 0 to 5.

2. The compound or the pharmaceutically acceptable salt thereof of claim 1, wherein, in Formula 1, R¹ and R² are each independently hydrogen or (Y)ₘSO₃⁻.

3. The compound or the pharmaceutically acceptable salt thereof of claim 1, wherein, in Formula 1, R³ and R⁴ are each independently (Y)ₙ(CO)R⁵;
R⁵ is a hydroxyl group or ; and
A is a C₂-C₆ heterocycloalkyl group containing any one or more heteroatoms selected from the group consisting of substituted or unsubstituted N, O, and S.

4. The compound or the pharmaceutically acceptable salt thereof of claim 3, wherein the A is

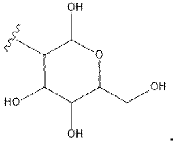

5. The compound or the pharmaceutically acceptable salt thereof of claim 1, wherein, in Formula 1, Z is Cl, 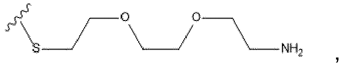 or

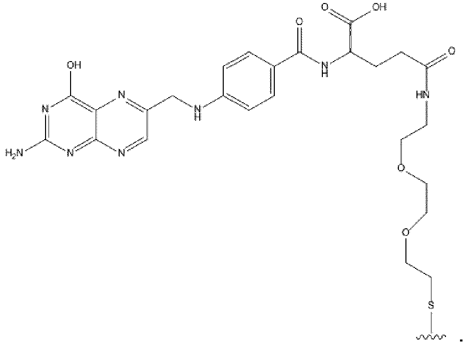

6. The compound or the pharmaceutically acceptable salt thereof of claim 1, wherein the compound is any one or more selected from the group consisting of compounds represented by the following [Formula 1-1] to [Formula 1-6]: 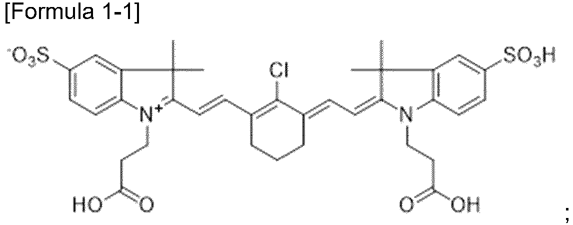 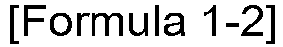 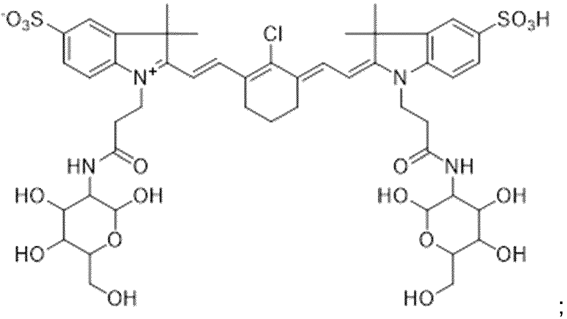 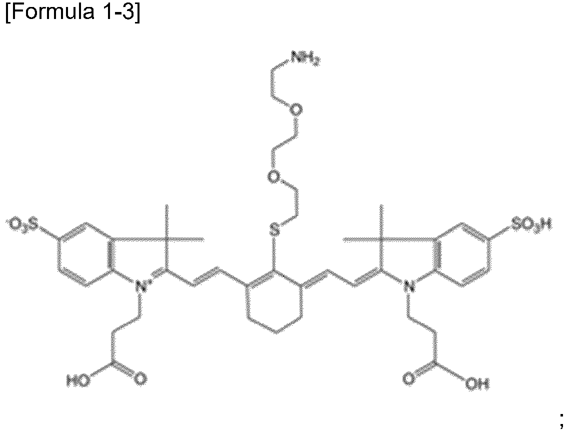 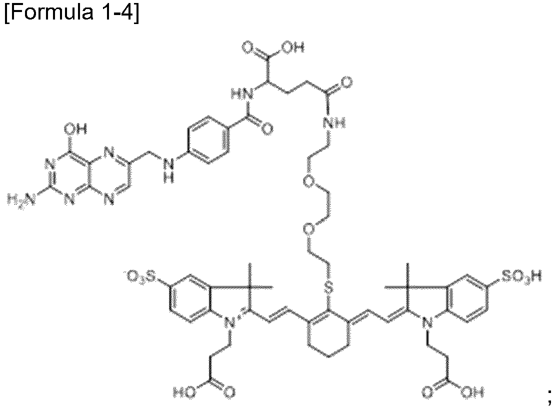 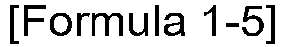 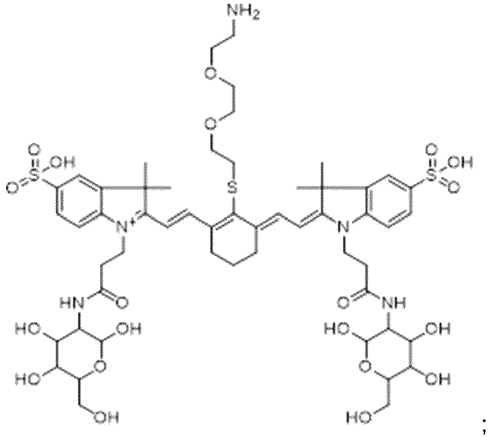 and

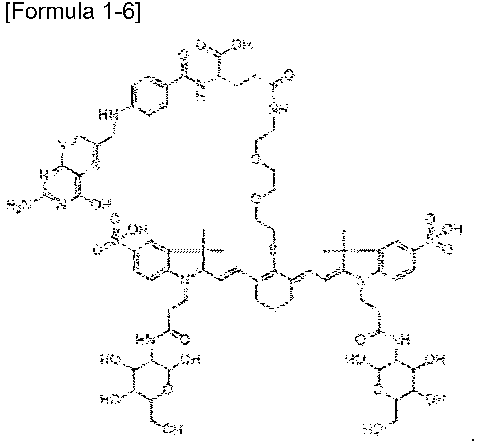

7. A contrast agent composition for cancer diagnosis comprising the compound of any one of claims 1 to 6 as an active ingredient.

8. The contrast agent composition of claim 7, wherein the contrast agent composition targets a tumor and/or tumor microenvironment.

9. The contrast agent composition of claim 7, wherein the contrast agent composition binds to any one or more of folate receptor alpha of cancer cells, folate receptor beta of tumor-associated macrophages (TAMs) in the tumor microenvironment, and CD206 of TAM.

10. The contrast agent composition of claim 7, wherein the contrast agent composition simultaneously targets a tumor and the tumor microenvironment.

11. A method for cancer diagnosis comprising targeting a tumor and/or tumor microenvironment using the composition of any one of claims 7 to 10.
